# EUROPEAN PATENT APPLICATION

(11) **EP 1 767 200 A1**
(43) Date of publication of application: **28.03.2007**
(21) Application number: 05751332.7
(22) Date of filing: 14.06.2005
(51) Int. Cl.: A61K 31/198, A61P 1/14, A61P 17/16, A61P 21/00, A61P 37/00, A61P 43/00

(54) **ANTIAGING AGENT**

(30) Priority: 15.06.2004 JP 2004177544
(71) Applicant: OSAKA PREFECTURAL GOVERNMENT, Osaka-shi Osaka 540-8570 (JP); Ajinomoto Co., Inc., Tokyo 104-8315 (JP)
(72) Inventor: TAKAGI, Yasuhiro, c/o Osaka Pref. Inst. of Pub. H., Osaka-shi, Osaka 5370025 (JP); AKIYAMA, Yukio, c/o Ajinomoto Co., Inc.,, Kawasaki-shi, Kanagawa 2108681 (JP); SHIBAHARA, Susumu, c/o Ajinomoto Co., Inc.,, Kawasaki-shi, Kanagawa 2108681 (JP)
(74) Representative: Nicholls, Kathryn Margaret
(86) International application number: PCT/JP2005/011200
(87) International publication number: WO 2005/123058

(57) **Abstract**

An effective anti-aging agent containing cystine and theanine in combination. Provision of a food or feed for anti-aging containing cystine and theanine in combination.

## Description

### Technical Field

The present invention relates to an anti-aging agent comprising cystine and theanine in combination, and a food or feed for anti-aging.

### Background Art

Aging is generally defined to be the decreased physiological function that occurs as we grow older. Specific examples of the physiological functions that are deteriorated as we grow old include basal metabolism, vital capacity, maximal breathing capacity, cardiac index, moisture content in the cell, nerve conduction velocity, renal glomerulus filtration rate, renal plasma flow, immune function and the like (IMAHORI Kazutomo, "What is aging?", Iwanami Shinsho, Iwanami Shoten, 2001). As we grow older, particularly in the elderly, morphological changes in the internal organs and muscles are observed, such as organ atrophy caused by decrease in the parenchymal cells of the organs, and skeletal muscle atrophy generally due to the lack of exercise resulting from the reduced amount of physical activity in the elderly. As a result, in the elderly, chronic diseases appear in the organs of the whole body, such as mental system, nervous system, circulatory system, respiratory system, gastrointestinal system, endocrine and metabolic system, hemoperfusion system, renal and urinary system, motor system, sensory system including skin and the like (SATO Kazuto, HONMA Takeshi, KOMATU Tatsushi ed., "Essential Clinical Nutrition Science", Ishiyaku Publishers, Inc., 2003).

Such chronic diseases degrade the quality of life (QOL) in the old age. For the improvement of QOL in the old age, therefore, it is important to improve or prevent aggravation of systemic condition that occurs as we grow old, such as deterioration of physiological function, decrease in the physical activity amount, morphological change in the internal organs and muscle, and the like.

It is described that the super system, homeostasis (maintenance of a constant state of living organism), wherein the cranial nerve system, the endocrine system and the immune system are related in complexity, declines as we grow older, which in turn degrades psychosomatic function, thus resulting in aging, and that anti-aging depends on how much the degradation of cranial nerve, endocrine and immune functions can be suppressed. However, it id only described that "nutrition" and "exercise" are effective for anti-aging, with no disclosure of specific anti-aging agents (IMAHORI Kazutomo, "What is Aging?", Iwanami Shinsho, Iwanami Shoten, 2001).

Here, theanine is an amino acid contained in green tea in a great amount. It has been disclosed that ethylamine produced after intake of theanine acts on the γδ type T cell, which is one kind of T cells, to promote production of interferon-γ (IFN-γ), a cytokine (A.B. Kamath et al., PNAS, 2003, vol. 100(10), 6009-6014). In addition, it has been disclosed that 4% of T cells in human peripheral blood is γδ type T cells, and theanine taken into the body is metabolized in the body to produce ethylamine, which acts on the γδT cells to promote production of interleukin-2 (IL-2), a cytokine (J.F. Bukowski et al., Immunity, 1999, vol. 11, 57-65). However, these actions were observed in vitro, and there is no report establishing that it in fact leads to the production of antibody in vivo. Moreover, there is no finding establishing that theanine intake actually promotes production of antibody in vivo.

Furthermore, no one has disclosed an anti-aging action, particularly, an improving or prophylactic action on the deteriorated physiological function and decreased immunity due to an increased age, by theanine or cystine, a sulfur amino acid, or a combination thereof.

### Disclosure of the Invention

The present invention aims at provision of an effective anti-aging agent, or a food or feed for anti-aging. As used in the present specification, the "anti-aging" means improvement or prophylaxis of degraded systemic conditions due to increased age, where the degraded systemic conditions due to increased age include decreased physiological functions (e.g., decreased immune function), a decreased amount of physical activity, morphological changes in the organs and muscle, and the like, which are due to increased age.

The present inventors have conducted intensive studies in an attempt to solve the above-mentioned problems and found that a combined administration of cystine and theanine has a superior immunoenhancing action on the aged mice, and a superior improving effect on the degraded systemic condition of the aged mice, which resulted in the completion of the present invention.

The present invention encompasses the following items.
(1) An anti-aging agent comprising cystine and theanine in combination.
(2) The anti-aging agent of the above-mentioned (1), wherein the aging is at least one selected from a decreased amount of exercise, decreased motor function, a decreased muscle force, atrophy of organ and skeletal muscle, decreased skin and hair moisturizing function and decreased immune function, which are due to increased age.
(3) A food or feed for anti-aging, comprising cystine and theanine in combination.
(4) The food or feed of the above-mentioned (3), wherein the aging is at least one selected from a decreased amount of exercise, decreased motor function, a decreased muscle force, atrophy of organ and skeletal muscle, decreased skin and hair moisturizing function, and decreased immune function, which are due to increased age.
(5) A commercial package comprising a combination agent comprising cystine and theanine in combination and a written matter associated therewith, the written matter stating that the combination agent can or should be used for anti-aging.
(6) The commercial package of the above-mentioned (5), wherein the aging is at least one selected from a decreased amount of exercise, decreased motor function, a decreased muscle force, atrophy of organ and skeletal muscle, decreased skin and hair moisturizing function, and decreased immune function, which are due to increased age.
(7) The anti-aging agent of the above-mentioned (1) or (2), wherein the cystine and theanine are combined in a weight ratio of 100:1 to 1:100.
(8) The anti-aging agent of the above-mentioned (1) or (2), wherein the dose of cystine and theanine in total is within the range of 100 µg/kg body weight to 800 mg/kg body weight per day.
(9) The anti-aging agent of the above-mentioned (1) or (2), wherein the dose of cystine is within the range of 1 µg/kg body weight to 800 mg/kg body weight per day.
(10) The anti-aging agent of the above-mentioned (1) or (2), wherein the dose of theanine is within the range of 1 µg/kg body weight to 800 mg/kg body weight per day.
(11) Use of cystine and theanine for the production of an anti-aging agent comprising cystine and theanine in combination.
(12) The use of (11), wherein the aging is at least one selected from a decreased amount of exercise, decreased motor function, a decreased muscle force, atrophy of organ and skeletal muscle, decreased skin and hair moisturizing function, and decreased immune function, which are due to increased age.
(13) Use of cystine and theanine for the production of a food or feed for anti-aging comprising cystine and theanine in combination.
(14) The use of (13), wherein the aging is at least one selected from a decreased amount of exercise, decreased motor function, a decreased muscle force, atrophy of organ and skeletal muscle, decreased skin and hair moisturizing function, and decreased immune function, which are due to increased age.
(15) The use of (11) or (12) for the production of an anti-aging agent, wherein the cystine and theanine are combined in a weight ratio of 100:1 to 1:100.
(16) The use of (11) or (12) for the production of an anti-aging agent, wherein the dose of cystine and theanine in total is within the range of 100 µg/kg body weight to 800 mg/kg body weight per day.
(17) The use of (11) or (12) for the production of an anti-aging agent, wherein the dose of cystine is within the range of 1 µg/kg body weight to 800 mg/kg body weight per day.
(18) The use of (11) or (12) for the production of an anti-aging agent, wherein the dose of theanine is within the range of 1 µg/kg body weight to 800 mg/kg body weight per day.
(19) A method of reducing the process of aging, comprising administering cystine and theanine in combination to a subject of administration.
(20) The method of (19), wherein the aging is at least one selected from a decreased amount of exercise, decreased motor function, a decreased muscle force, atrophy of organ and skeletal muscle, decreased skin and hair moisturizing function, and decreased immune function, which are due to increased age.
(21) A method of reducing the process of aging, comprising administering a food or feed comprising cystine and the anine in combination to a subject of administration.
(22) The method of (21), wherein the aging is at least one selected from a decreased amount of exercise, decreased motor function, a decreased muscle force, atrophy of organ and skeletal muscle, decreased skin and hair moisturizing function, and decreased immune function, which are due to increased age.
(23) The method of (19) or (20), wherein the cystine and theanine are combined in a weight ratio of 100:1 to 1:100.
(24) The method of the above-mentioned (19) or (20), wherein the dose of cystine and theanine in total is within the range of 100 µg/kg body weight to 800 mg/kg body weight per day.
(25) The method of the above-mentioned (19) or (20), wherein the dose of cystine is within the range of 1 µg/kg body weight to 800 mg/kg body weight per day.
(26) The method of the above-mentioned (19) or (20), wherein the dose of the anine is within the range of 1 µg/kg body weight to 800 mg/kg body weight per day.

The anti-aging agent and the food or feed for anti-aging of the present invention can improve or prevent degraded systemic conditions due to increased age (e.g., decreased physiological function, morphological changes of organ and muscle, decreased amount of physical activity and the like, specifically, for example, a decreased amount of exercise, decreased motor function (e.g., speed during walking and running, jumping power, endurance, instantaneous force etc.), a decreased muscle force (particularly decreased tightness of muscle), atrophy of organ and skeletal muscle, decreased moisturizing function of skin and hair/body hair or feather (e.g., dry skin, hair/body hair or feather, low skin elasticity, decreased gloss of skin, hair/body hair or feather, etc.), decreased immune function, decreased daily activity and the like).

### Best Mode for Embodying the Invention

The cystine used in the present invention is preferably L-cystine.

The cystine may be in the form of a salt, and the term "cystine" used in the present specification conceptually also encompasses salts.

Such salt is not particularly limited as long as it is pharmacologically acceptable and, for example, salts with inorganic acid or organic acid can be mentioned. As the inorganic acid, for example, hydrochloric acid, hydrobromic acid, nitric acid, sulfuric acid, phosphoric acid and the like can be mentioned, and as the organic acid, formic acid, acetic acid, trifluoroacetic acid, propionic acid, lactic acid, tartaric acid, oxalic acid, fumaric acid, maleic acid, citric acid, malonic acid, methanesulfonic acid and the like can be mentioned. In addition, it can be a salt with a base. As the salt with a base, for example, alkali metal salts such as sodium salt, potassium salt and the like, alkaline earth metal salts such as calcium salt, magnesium salt and the like, and the like can be mentioned.

The theanine to be used in the present invention is preferably L-theanine.

The theanine may take the form of a salt and the term "the anine" in the present specification conceptually also encompasses salts.

Such salt is not particularly limited as long as it is pharmacologically acceptable, and, for example, salts with inorganic acid or organic acid can be mentioned. As the inorganic acid, for example, hydrochloric acid, hydrobromic acid, nitric acid, sulfuric acid, phosphoric acid and the like can be mentioned, and as the organic acid, formic acid, acetic acid, trifluoroacetic acid, propionic acid, lactic acid, tartaric acid, oxalic acid, fumaric acid, maleic acid, citric acid, malonic acid, methanesulfonic acid and the like can be mentioned. In addition, it can be a salt with a base. As the salt with a base, for example, alkali metal salts such as sodium salt, potassium salt and the like, alkaline earth metal salts such as calcium salt, magnesium salt and the like, and the like can be mentioned.

The anti-aging agent of the present invention can be advantageously applied to human and animals other than human [e.g., mammals other than human (domestic animals and pets such as pig, bovine, horse, dog and the like), birds (poultry and pets such as turkey, chicken and the like) and the like], and the like.

The anti-aging agent of the present invention includes cystine and theanine in combination (i.e., combination agent), and only need to be able to combine cystine with theanine on administration. Accordingly, the anti-aging agent of the present invention may be a single preparation obtained by simultaneously formulating cystine and theanine into a preparation or a combination of two kinds of preparations obtained by separately formulating cystine and theanine into preparations, as long as cystine with theanine can be combined on administration. The mode of administration is not particularly limited and, for example, (1) administration as a composition containing cystine and theanine, namely, a single preparation, (2) simultaneous administration of 2 kinds of preparations obtained by separately formulating cystine and theanine into preparations by the same administration route, (3) administration of 2 kinds of preparations obtained by separately formulating cystine and theanine into preparations by the same administration route at staggered times (e.g., administration in the order of cystine, then theanine, or in the reverse order), (4) simultaneous administration of 2 kinds of preparations obtained by separately formulating cystine and theanine into preparations by different administration routes, (5) administration of 2 kinds of preparations obtained by separately formulating cystine and theanine into preparations by different administration routes at staggered times (e.g., administration in the order of cystine, then theanine, or in the reverse order) and the like can be mentioned.

In the case of administration at staggered times, it is necessary that the both be co-present in the body.

In the anti-aging agent of the present invention, the ratio of the combination of cystine and theanine is, whether they are processed into a single preparation or separate preparations, generally in the range of 100:1 - 1:100, preferably in the range of 20:1 - 1:2, by weight ratio.

In the anti-aging agent of the present invention, the total daily dose of cystine and theanine is generally in the range of 100 µg/kg body weight - 800 mg/kg body weight, preferably in the range of 500 µg/kg body weight - 100 mg/kg body weight. However, the daily dose of cystine is generally in the range of 1 µg/kg body weight - 800 mg/kg body weight, preferably in the range of 100 µg/kg body weight - 100 mg/kg body weight.

Moreover, the daily dose of theanine is generally in the range of 1 µg/kg body weight - 800 mg/kg body weight, preferably in the range of 20 µg/kg body weight - 100 mg/kg body weight.

In the anti-aging agent of the present invention, the above-mentioned daily dose can be administered once a day or in several portions a day. While the administration period is not particularly limited.

The dosage form of the anti-aging agent of the present invention is not particularly limited, and may be any of oral preparation and parenteral preparation. As the dosage form, for example, tablet, granule, powder, capsule, elixir, syrup, microcapsule or suspension and the like can be mentioned. The anti-aging agent of the present invention can be orally or parenterally applied.

For parenteral administration, for example, a solution containing cystine and theanine can be administered as a nasal spray or injection, and the like. The oral administration can be given any time, for example, before meals, after meals and between meals.

The anti-aging agent of the present invention can contain carrier, excipient, binder, swelling agent, lubricant, sweetener, flavor, preservative, stabilizer, coating agent and the like, as necessary, and can be used in a unit dose form requested for conventionally established practice of preparation. The amounts of cystine and theanine in these compositions or preparations may be such doses that afford suitable dose in the specified range.

In the anti-aging agent of the present invention, as specific components that can be contained in, for example, binders such as tragacanth, gum arabic, corn starch and gelatin; excipients such as micro-crystalline cellulose and crystalline cellulose; swelling agents such as corn starch, pregelatinized starch, alginic acid and dextrin; lubricants such as magnesium stearate; flowability improvers such as silicon dioxide microparticle and methylcellulose; gloss agents such as glycerin fatty acid ester; sweeteners such as sucrose, lactose and aspartam; flavors such as peppermint, vanilla flavoring and cherry or orange flavoring; stabilizers such as monoglyceride, polyglycerol esters of fatty acid, sucrose esters of fatty acid and lecithin; and the like can be mentioned.

When the unit dosage form is a capsule, a liquid carrier such as fat can be further contained in the abode-mentioned type of materials.

In addition, various other materials can be contained for changing the physical form of the unit dosage form. As the coating agent for tablet, for example, shellac, sugar or both of them can be mentioned. Syrup and elixir can contain, for example, sucrose as a sweetener, and methylparaben and propylparaben as preservatives, dye and cherry or orange flavor and the like. In addition, various vitamins and various amino acids may be contained.

When an enteric-coated preparation is produced, for example, an aqueous solution of hydroxyphenyl methyl cellulose is used as a pre-coating treating agent, and an aqueous solution of hydroxypropylmethylcellulose phthalate and an aqueous solution of polyacetin are used as coating agents to give an enteric-coated preparation by a conventional method.

The anti-aging agent of the present invention can be used for improving or preventing degraded systemic conditions due to increased age (e.g., decreased physiological function, morphological changes of organ and muscle, decreased amount of physical activity and the like, specifically, for example, a decreased amount of exercise, decreased motor function (e.g., speed during walking and running, jumping power, endurance, instantaneous force etc.), a decreased muscle force (particularly decreased tightness of muscle), atrophy of organ and skeletal muscle, decreased moisturizing function of skin and hair/body hair or feather (e.g., dry skin, hair/body hair or feather, low skin elasticity, decreased gloss of skin and hair/body hair or feather, etc.), decreased immune function, decreased daily activity and the like).

The present invention encompasses a commercial package comprising a combination agent comprising cystine and theanine in combination, and a written matter associated therewith, the written matter stating that the combination agent can or should be used for anti-aging.

The food for anti-aging in the present invention comprises cystine and theanine in combination, and may be any as long as cystine and theanine can be combined when eating or drinking. The amount of cystine and theanine contained in the food for anti-aging of the present invention is not particularly limited, but preferably the amount drunk or eaten a day is in the same range as the above-mentioned dose of the anti-aging agent of the present invention. While the form of the food for anti-aging of the present invention is not particularly limited, for example, one wherein cystine and theanine are added to food such as a beverage base powder (e.g., milk powder and the like), beverage and confectionery and the like can be mentioned.

The "food" for anti-aging in the present invention means food in general, which includes general food including what is called health food, as well as food for specified health use and food with nutrient function claims, specified in the Food with health claims system by the Japanese Ministry of Health, Labour and Welfare, further including dietary supplements. In addition, the anti-aging agent of the present invention can be applied to feed use, and can be administered to poultry, domestic animals and the like by addition to general feed.

### Examples

The present invention is explained in detail in the following by referring to Examples, which are not to be construed as limitative.

### Example 1

To 2-year-old aged mice (C3H/HeN, female) were orally administered a mixture of cystine and the anine (10:4, weight ratio), which had been dissolved in distilled water to 16 mg/kg body weight (based on human adult dose 980 mg/60 kg body weight/day) and 32 mg/kg body weight, once a day for 2 weeks with a stomach tube. The liquid dose per administration was 0.1 mL per 25 g body weight. The control group was administered with deionized water.

On the next day of the final administration, 200 µg of DNP-Dextran as an antigen was administered to one mouse via the tail vein for antigen stimulation. At 4, 6 and 10 days after the antigen stimulation, blood samples were collected from the subclavian vein, and the production amount of the anti-DNP-IgM antibody in the serum was measured by ELISA, and the effect of cystine/theanine on the IgM antibody production was examined.

The measurement method of the antibody amount by ELISA is as follows.

DNP-BSA (2 µg/mL) was dispensed to a 96-well microplate (Immulon2 manufactured by Dynatech) at 100 µL/well, and coated by incubation at 37°C for 2-3 hr.

After washing with 0.05% Tween 20-PBS, a sample was added at 100 µL/well, and the mixture was stood at 4°C for about 18 hr. After washing, an enzyme labeled antibody (HRP-rabbit anti-mouse IgM) was added at 100 µL/well, and the mixture was incubated at 37°C for 60 min. After washing, a substrate solution (100 µL/well) was added, and the mixture was reacted at room temperature for about 10 min with light shielding, after which 2N sulfuric acid (100 µL/well) was added to stop the reaction. The OD value was measured using a micro-plate reader (wavelength 490 nm). The sample dilution rate showing an OD value of 0.5 was taken as the antibody amount.

The antibody amount of each individual is shown in Table 1. From Table 1, the antibody amounts at 4, 6 and 10 days after the antigen administration were generally in the range of 10,000-21,000 in the control group (5 mice), whereas the highest antibody amounts of 23,000-30,000 were found in 3 cases out of 5 cases of the cystine/theanine 16 mg/kg body weight administration group and in 3 cases out of 4 cases of the 32 mg/kg body weight administration group, and a clear increase in the IgM antibody production amount in the aged mice due to the cystine/theanine administration was confirmed.

**Table 1 Effect of cystine/theanine on IgM antibody-producing ability in aged mice (individual value)**

| | Individual No. | 4 days later | 6 days later | 10 days later |
|---|---|---|---|---|
| control group | 1 | 13600 | 21152 | 12735 |
| | 2 | 10667 | 16271 | 19633 |
| | 3 | 19294 | 18967 | 18436 |
| | 4 | 18627 | 19849 | 18791 |
| | 5 | 12493 | 17493 | 19200 |
| cystine/theanine 16 mg/kg body weight administration group | 1 | 23318 | 28160 | 28009 |
| | 2 | 22652 | 23930 | 22970 |
| | 3 | 5311 | 8727 | 10504 |
| | 4 | 12697 | 13357 | 18786 |
| | 5 | 24219 | 27014 | 19319 |
| cystine/theanine 32 mg/kg body weight administration group | 1 | 9964 | 11749 | 10962 |
| | 2 | 29275 | 31226 | 16559 |
| | 3 | 11439 | 12149 | 23909 |
| | 4 | 25831 | 26344 | 27254 |

| | | | | |
|---|---|---|---|---|
| The numeral values are IgM values (antibody amounts). | | | | |

An average IgM value (antibody amount) of each group is shown in Table 2. From the result, it has been clarified that IgM antibody production is potentiated from the early stage of 4 days after the antigen administration in the cystine/theanine administration group, and cystine/theanine is useful for decreased immune function due to increased age.

It has been reported that, in aged mice (about 2 years old), the IgM antibody producing ability decreases to about 35% as compared to that in mature mice (propagation suitable stage, about 3 months old) (TAKAGI Yasuhiro et al., Journal of Traditional Medicines 13, 94, 1996).

**Table 2 Effect of cystine/theanine on IgM antibody-producing ability in aged mice (average value)**

| | 4 days later | 6 days later | 10 days later |
|---|---|---|---|
| control group | 15270 | 18747 | 17759 |
| cystine/theanine 16 mg/kg body weight administration group | 17639 | 20238 | 19917 |
| cystine/theanine 32 mg/kg body weight administration group | 19127 | 20367 | 19671 |

| | | | |
|---|---|---|---|
| The numeral values are IgM values (antibody amounts). | | | |

### Example 2

In the same manner as in Example 1, a mixture of cystine and theanine and deionized water as a control were administered to 2-year-old aged mice. Thereafter, 100 µg of DNP-KLH and Freund's incomplete adjuvant were mixed to give a water-in-oil suspension, which was intraperitoneally injected for primary antigen stimulation. For secondary antigen stimulation 4 weeks later, 50 µg of DNP-KLH (PBS solution) was intraperitoneally injected. After the secondary antigen stimulation, blood samples were collected from the jugular vein of the mice 10 days later. Serum was separated from the blood samples, and the anti-DNP-IgG antibody amount in the serum was measured in the same manner as in ELISA of Example 1. In this case, an HRP-rabbit anti-mouse IgG antibody was used as the enzyme labeled antibody. The sample dilution rate showing an OD value of 0.5 was taken as the antibody amount. The antibody amount of each individuals is shown in Table 3.

**Table 3 Effect of cystine/theanine on IgM antibody-producing ability in aged mice (individuals value)**

| | Individual No. | 10 days later (×10⁴) |
|---|---|---|
| control group | 1 | 57.6 |
| | 2 | 40.2 |
| | 3 | 28.6 |
| | 4 | 35.4 |
| | 5 | 50.4 |
| | 6 | 20.5 |
| cystine/theanine 16 mg/kg body weight administration group | 1 | 60.6 |
| | 2 | 60.6 |
| | 3 | 88.6 |
| | 4 | 82.5 |
| | 5 | 73.9 |
| | 6 | 67.3 |
| cystine/theanine 32 mg/kg body weight administration group | 1 | 36.5 |
| | 2 | 49.5 |
| | 3 | 80.0 |
| | 4 | 78.9 |
| | 5 | 73.8 |
| | 6 | 48.2 |

| | | |
|---|---|---|
| The numeral values are IgM values (antibody amounts). | | |

From Table 3, the average IgG value (antibody amount) of each group is 38.8 (×10⁴) for the control group, 70.2(×10⁴) for the cystine/theanine 16 mg/kg body weight administration group, and 61.2(×10⁴) for the cystine/theanine 32 mg/kg body weight administration group, and the cystine/theanine administration group has been clarified to potentiate the IgG antibody production by the administration before the primary antigen stimulation. When the control group is 100%, the production was enhanced by 181% in the cystine/theanine 16 mg/kg body weight administration group, and 158% in the 32 mg/kg body weight administration group, and cystine/theanine was shown to be useful for the decreased immune function due to the increased age.

According to Takagi et al., when the IgG antibody titer of the mature mouse (propagation suitable stage, about 3-month-old) is 100, it has been clarified that the IgG antibody producing ability gradually decreases due to increased age, as evidenced by 56 for 6 month old and 38 for one year old (TAKAGI Yasuhiro et al., Osaka Prefectural Institute of Public Health report, vol. 40, p. 19, 2002).

### Example 3

To clarify the effect of the cystine/theanine administration on the degraded systemic conditions due to increased age, aged mice were used for investigation.

In the same manner as in Example 1, 2-year-old aged mice (C3H/HeN, female) were orally administered a mixture of cystine and theanine (10:4, weight ratio), which had been dissolved in distilled water to 16 mg/kg body weight (based on human adult dose 980 mg/60 kg body weight/day) and 32 mg/kg body weight, once a day for 2 weeks with a stomach tube. The liquid dose per administration was 0.1 mL per 25 g body weight. The control group was administered with deionized water.

The results observed from 3 days after the administration are shown in Table 4. In Table 4, "-" shows a smaller amount of exercise, no tightness of muscle, no gloss of body hair, low skin moisturization or low skin elasticity as compared to mature mouse (propagation suitable stage, about 3 month old), "+" shows a greater amount of exercise, tightness of muscle, gloss of body hair, high skin moisturization or high skin elasticity as compared to the control group (from 3 days after administration). In Table 4, "-" means "-" for the item in all cases of the group, and "+" means "+" for the item in not less than half cases of the group.

As is clear from Table 4, by the administration of cystine/theanine (16 mg/kg body weight and 32 mg/kg body weight), a clear physical condition-improving effect on the aged mice as evidenced by the increased amount of exercise, increased tightness of muscle, increased gloss of body hair, increased skin moisturization, increased skin elasticity and the like was observed from 3 days after the administration.

**Table 4 Effect of cystine/theanine on degraded systemic conditions in aged mice**

| observation item | control group (5 cases) | cystine/theanine 16 mg/kg body weight administration group (5 cases) | cystine/theanine 32 mg/kg body weight administration group (4 cases) |
|---|---|---|---|
| amount of exercise | - | + | + |
| tightness of muscle | - | + | + |
| body hair gloss | - | + | + |
| moisturization of skin | - | + | + |
| skin elasticity | - | + | + |

From the above results, it has been shown that cystine/theanine is useful for the degraded systemic conditions due to increased age (decreased amount of exercise, decreased muscle force, decreased skin and hair moisturizing function, decreased immune function etc.), in addition to the immunoenhancing action observed in Example 1 and Example 2.

### Industrial Applicability

Since cystine and theanine, which are the active ingredients of the anti-aging agent of the present invention, are substances having established safety, the anti-aging agent of the present invention has high safety and therefore can be used not only for pharmaceutical use but also food and feed.

While some of the embodiments of the present invention have been described in detail in the above, it will, however, be evident for those of ordinary skill in the art that various modifications and changes may be made to the particular embodiments shown without substantially departing from the novel teaching and advantages of the present invention. Such modifications and changes are encompassed in the spirit and scope of the present invention as set forth in the appended claims.

This application is based on application No. 2004-177544 (filing date: June 15, 2004) filed in Japan, the contents of which are incorporated hereinto by reference.

## Claims

1. An anti-aging agent comprising cystine and theanine in combination.

2. The anti-aging agent of claim 1, wherein the aging is at least one selected from a decreased amount of exercise, decreased motor function, a decreased muscle force, atrophy of organ and skeletal muscle, decreased skin and hair moisturizing function and decreased immune function, which are due to increased age.

3. A food or feed for anti-aging, comprising cystine and the anine in combination.

4. The food or feed of claim 3, wherein the aging is at least one selected from a decreased amount of exercise, decreased motor function, a decreased muscle force, atrophy of organ and skeletal muscle, decreased skin and hair moisturizing function, and decreased immune function, which are due to increased age.

5. A commercial package comprising a combination agent comprising cystine and theanine in combination and a written matter associated therewith, the written matter stating that the combination agent can or should be used for anti-aging.

6. The commercial package of claim 5, wherein the aging is at least one selected from a decreased amount of exercise, decreased motor function, a decreased muscle force, atrophy of organ and skeletal muscle, decreased skin and hair moisturizing function, and decreased immune function, which are due to increased age.

7. The anti-aging agent of claim 1 or 2, wherein the cystine and theanine are combined in a weight ratio of 100:1 to 1:100.

8. The anti-aging agent of claim 1 or 2, wherein the dose of cystine and theanine in total is within the range of 100 µg/kg body weight to 800 mg/kg body weight per day.

9. The anti-aging agent of claim 1 or 2, wherein the dose of cystine is within the range of 1 µg/kg body weight to 800 mg/kg body weight per day.

10. The anti-aging agent of claim 1 or 2, wherein the dose of theanine is within the range of 1 µg/kg body weight to 800 mg/kg body weight per day.

11. Use of cystine and the anine for the production of an anti-aging agent comprising cystine and theanine in combination.

12. The use of claim 11, wherein the aging is at least one selected from a decreased amount of exercise, decreased motor function, a decreased muscle force, atrophy of organ and skeletal muscle, decreased skin and hair moisturizing function, and decreased immune function, which are due to increased age.

13. Use of cystine and theanine for the production of a food or feed for anti-aging comprising cystine and theanine in combination.

14. The use of claim 13, wherein the aging is at least one selected from a decreased amount of exercise, decreased motor function, a decreased muscle force, atrophy of organ and skeletal muscle, decreased skin and hair moisturizing function, and decreased immune function, which are due to increased age.

15. The use of claim 11 or 12 for the production of an anti-aging agent, wherein the cystine and theanine are combined in a weight ratio of 100:1 to 1:100.

16. The use of claim 11 or 12 for the production of an anti-aging agent, wherein the dose of cystine and theanine in total is within the range of 100 µg/kg body weight to 800 mg/kg body weight per day.

17. The use of claim 11 or 12 for the production of an anti-aging agent, wherein the dose of cystine is within the range of 1 µg/kg body weight to 800 mg/kg body weight per day.

18. The use of claim 11 or 12 for the production of an anti-aging agent, wherein the dose of the anine is within the range of 1 µg/kg body weight to 800 mg/kg body weight per day.

19. A method of reducing the process of aging, comprising administering cystine and theanine in combination to a subject of administration.

20. The method of claim 19, wherein the aging is at least one selected from a decreased amount of exercise, decreased motor function, a decreased muscle force, atrophy of organ and skeletal muscle, decreased skin and hair moisturizing function, and decreased immune function, which are due to increased age.

21. A method of reducing the process of aging, comprising administering a food or feed comprising cystine and theanine in combination to a subject of administration.

22. The method of claim 21, wherein the aging is at least one selected from a decreased amount of exercise, decreased motor function, a decreased muscle force, atrophy of organ and skeletal muscle, decreased skin and hair moisturizing function, and decreased immune function, which are due to increased age.

23. The method of claim 19 or 20, wherein the cystine and theanine are combined in a weight ratio of 100:1 to 1:100.

24. The method of claim 19 or 20, wherein the dose of cystine and theanine in total is within the range of 100 µg/kg body weight to 800 mg/kg body weight per day.

25. The method of claim 19 or 20, wherein the dose of cystine is within the range of 1 µg/kg body weight to 800 mg/kg body weight per day.

26. The method of claim 19 or 20, wherein the dose of theanine is within the range of 1 µg/kg body weight to 800 mg/kg body weight per day.
